# EUROPÄISCHE PATENTSCHRIFT

(11) **EP 1 872 706 B1**
(45) Veröffentlichungstag und Bekanntmachung des Hinweises auf die Patenterteilung: **12.09.2012**
(21) Anmeldenummer: 07012791.5
(22) Anmeldetag: 29.06.2007
(51) Int. Cl.: A61B 1/00, G02B 23/24

(54) **Verfahren zum Herstellen eines Endoskops sowie derartiges Endoskop**
Method for manufacturing an endoscope and endoscope
Procédé destiné à la fabrication d'un endoscope tout comme endoscope de ce type

(30) Priorität: 01.07.2006 DE 102006030521
(43) Veröffentlichungstag der Anmeldung: 02.01.2008
(73) Patentinhaber: Karl Storz GmbH & Co. KG, 78532 Tuttlingen (DE)
(72) Erfinder: Salvermoser, Markus, 78532 Tuttlingen (DE); Stihl, Ewald, 78187 Geisingen (DE)
(74) Vertreter: Heuckeroth, Volker

(56) Entgegenhaltungen:
- DE-A1- 19 713 275
- JP-A- 58 097 439
- US-A- 2 621 651
- US-A- 4 237 871
- US-A- 5 341 566
- US-A1- 2006 069 307

## Beschreibung

Die Erfindung betrifft ein Verfahren zum Herstellen eines Endoskops, wobei das Endoskop einen Endoskopkopf und ein Optikrohr aufweist, mit den Schritten Bereitstellen des Endoskopkopfs, Bereitstellen des Optikrohrs und Einschieben eines proximalen Endbereichs des Optikrohrs in einen distalen Endbereich des Endoskopkopfs.

Die Erfindung betrifft ferner ein Endoskop mit einem Endoskopkopf und einem Optikrohr.

Ein derartiges Verfahren sowie ein Endoskop, das durch dieses Verfahren hergestellt ist, sind aus DE 197 13 275 A1 bekannt.

Endoskope werden vor allem in der minimal-invasiven Chirurgie verwendet, um Körperhohlräume oder Hohlorgane zu untersuchen. Hierzu weist ein Endoskop einen Endoskopkopf auf, an dessen distalen Ende ein langerstreckter Endoskopschaft angeordnet ist, der ein Optikrohr aufweist. Am Endoskopkopf können Anschlüsse für eine externe Beleuchtungsquelle und Spül-/Saugleitungen sowie eine Augenmuschel vorgesehen sein. Es wird über den Anschluss für die Beleuchtungsquelle Licht in ein Lichtleitsystem, das durch den Endoskopkopf und den Endoskopschaft reicht, eingekoppelt und zu dem distalen Ende des Endoskopschafts geführt. Ferner weist das Endoskop eine Optik zur Bildgebung auf. Die Bildgebungsoptik kann aus Lichtleitfasern, Stablinsen o.a. gefertigt sein, aber auch einen Bildsensor mit elektrischer Signalübertragung aufweisen.

Endoskope können je nach Material des Endoskopschafts starr oder flexibel ausgebildet sein. Ein starres Endoskop weist einen Schaft aus einem nicht biegsamen Material, wie bspw. Edelstahl oder Metall, auf.

Zur Untersuchung eines Körperhohlraums wird ein distaler Endbereich des Endoskops in diesen eingeführt, während der Endoskopkopf und der Teil des Endoskopschafts, der nicht in den Körperhohlraum eingeführt ist, außerhalb des Körpers verbleibt.

Die Herstellung eines Endoskops umfasst unter anderem das Verbinden eines proximalen Endbereichs des Schafts mit einem distalen Endbereich des Endoskopkopfs.

Bekannte Verfahren gehen davon aus, den Endoskopkopf und das Optikrohr des Endoskops bereitzustellen und beide derart ineinander einzuschieben, dass das Optikrohr mit seinem proximalen Endbereich in dem distalen Endbereich des Endoskopkopfs aufgenommen ist. Die Berührflächen beider Endbereiche werden durch Löten, Schweißen oder Verkleben miteinander verbunden, wie in dem oben genannten Dokument DE 197 13 275 A1 in Bezug auf Löten offenbart ist.

Nachteilig an diesen bekannten Verfahren ist, dass das Verbinden des Endoskopkopfs und des Optikrohrs technisch sehr aufwändig ist. Im Fertigungsprozess des Endoskops müssen entsprechende Maschinen bereitgestellt werden, die ein Verschweißen, Verlöten oder verkleben der Berührflächen des Endoskopkopfs und des Optikrohrs ermöglichen. Ferner sind diese bekannten Verfahren, den Endoskopkopf mit dem Optikrohr zu verbinden, sehr zeitaufwändig. Insbesondere muss beim Verkleben beider Endoskopbauteile darauf geachtet werden, dass das Optikrohr und der Endoskopkopf so lange lagefest gehalten werden, bis der Klebstoff getrocknet ist.

Ein weiterer Nachteil dieser Verfahren ist die Schwierigkeit, das Optikrohr und den Endoskopkopf beim Herstellungsprozess zueinander exakt positioniert zu halten, so dass die Länge des Endoskops reproduzierbar ist. Weicht die sich ergebende Gesamtlänge des hergestellten Endoskops minimal von der gewünschten Solllänge ab, muss dieser Längenunterschied beim Ausbilden des Lichtleitsystems und der Bildgebungsoptik berücksichtigt werden.

Ferner ist es bei den bekannten Herstellungsverfahren mittels Schweißen und Löten nachteilig, dass während des Herstellungsprozesses Wärme im Bereich der Verbindungsstelle beider Endoskopbauteile entsteht. Durch die Erwärmung des Materials des Optikrohrs kann dieses spröde werden, wodurch die Festigkeit des Optikrohrs im Bereich der Wärmeinwirkung herabgesetzt wird, so dass das Optikrohr bereits bei leichten Biegebeanspruchungen brechen kann,

Ferner erweist es sich als Nachteil, dass bei Endoskopen, die mittels der bekannten Verfahren hergestellt sind, die Verbindung zwischen dem Endoskopkopf und dem Optikrohr nicht stabil ausgebildet ist. Wird das Optikrohr während einer Operation infolge einer Biegebeanspruchung einer starken Hebelkraft ausgesetzt, ist es möglich, dass das Endoskop an der Verbindungsstelle, d.h. der Sch`weiß-, Löt- oder Klebestelle, zwischen dem Endoskopkopf und dem Optikrohr bricht.

US-A-2 621 651 offenbart ein Instrument zum Aufweiten und Untersuchen der Speiseröhre. Das Instrument weist einen Außenschaft auf, der ein Endoskop aufnimmt, das einen Endoskopkopf und ein Optikrohr aufweist. Das proximale Ende des Außenschafts ist in eine Öffnung einer Buchse eingeführt und mit der Buchse verpresst.

US 2006/0069307 A1 offenbart eine Schaftkupplung, mit der ein Endoskopschaft an einem Gehäuse drehbar befestigt werden kann.

US-A-4 237 871 offenbart ein medizinisches Instrument zum Injizieren von Pasten oder Fluiden in den menschlichen Körper. Dieses Instrument weist einen Schaft für die Aufnahme des Optikrohrs eines Endoskops auf. Ein Injektionsrohr dieses Instruments, das parallel zu und seitlich von dem Schaft zur Aufnahme des Optikrohrs angeordnet ist, ist mit dem Instrumentenkopf fest verbunden.

Es ist eine Aufgabe der vorliegenden Erfindung, den oben genannten Nachteilen Abhilfe zu schaffen und ein Verfahren der eingangs genannten Art bereitzustellen, das eine technisch einfach realisierbare und stabile Verbindung zwischen dem Endoskopkopf und dem Optikrohr ermöglicht.

Es ist ferner eine Aufgabe der vorliegenden Erfindung, ein Endoskop der eingangs genannten Art bereitzustellen, bei dem der Endoskopkopf konstruktiv einfach und stabil mit dem Optikrohr verbunden ist.

Erfindungsgemäß wird die Aufgabe hinsichtlich des eingangs genannten Verfahrens dadurch gelöst, dass der distale Endbereich des Endoskopkopfs mit dem proximalen Endbereich des Optikrohrs verpresst wird, und dass der distale Endbereich des Endoskopkopfs innenseitig mit einem umfänglichen Einstich bereitgestellt wird, in den beim Verpressen Material des Optikrohrs formschlüssig einfließt.

Des Weiteren wird die Aufgabe hinsichtlich des eingangs genannten Endoskops erfindungsgemäß dadurch gelöst, dass ein proximaler Endbereich des Optikrohrs mit einem distalen Endbereich des Endoskopkopfs verpresst ist, und dass der distale Endbereich des Endoskopkopfs innenseitig einen umfänglichen Einstich aufweist, in den Material des Optikrohrs formschlüssig eingreift.

Das erfindungsgemäße Verfahren und das erfindungsgemäße Endoskop ermöglichen eine technisch sehr einfache Verbindung zwischen dem Endoskopkopf und dem Optikrohr. Ein Verpressen der beiden Endoskopbauteile bedarf vorteilhafterweise keiner weiteren Hilfsmittel, wie z.B. Lötzinn oder Klebstoff. Ferner kann das erfindungsgemäße Verfahren zeitsparender als ein Verlöten, Verschweißen oder Verkleben des Endoskopkopfs mit dem Optikrohr durchgeführt werden. Daher entfallen bei der Fertigung des Endoskops Prozessschritte wie Abkühlen oder Trocknen der Verbindurigsstelle zwischen dem Endoskopkopf und dem Optikrohr.

Durch das Verpressen des Optikrohrs mit dem Endoskopkopf, das kalt bzw. ohne Erwärmung durchgeführt werden kann, entstehen auch keine Materialschwächungen durch Wärmeeinflüsse, wie dies beim Schweißen oder Löten der Fall ist.

Ein weiterer Vorteil des erfindungsgemäßen Verfahrens gegenüber den bekannten Verfahren ergibt sich aus der Tatsache, dass die Verbindung zwischen dem Optikrohr und dem Endoskopkopf stabil und zeitlich beständig ausgebildet ist. Da beide Endoskopbauteile miteinander verpresst sind, ist das Endoskop gegenüber äußeren Einflüssen, wie zum Beispiel Wasser, unempfindlich. Folglich lockert sich das Optikrohr auch nicht nach vielen Reinigungsprozessen des Endoskops, so dass kostenintensive Reparaturen vermieden werden.

Ferner ermöglicht das Verpressen des Endoskopkopfs mit dem Optikrohr eine stabile Verbindung beider Bauteile. Hierdurch hält das Endoskop vorteilhafterweise auch starken Biegebeanspruchungen des Optikrohrs stand, die eine große Hebelkraft auf das Optikrohr erzeugen.

Dadurch dass der distale Endbereich des Endoskopkopfs innenseitig mit einem umfänglichen Einstich bereitgestellt wird, in den beim Verpressen Material des Optikrohrs formschlüssig einfließt, wird der Vorteil erzielt, dass das Optikrohr in dem Endoskopkopf axial besonders sicher gehalten wird, indem Material des Optikrohrs in den Einstich des Endoskopkopfs eingreift. Zug- und Druckkräfte auf das Optikrohr in axialer Richtung führen nicht zu einer Lageänderung des Optikrohrs bezüglich des Endoskopkopfs.

In einer bevorzugten Ausgestaltung wird der proximale Endbereich des Optikrohrs durch Stauchen verpresst.

Diese Maßnahme stellt eine konstruktiv einfach zu realisierende Verpressung des Optikrohrs mit dem Endoskopkopf dar. Da die Wandstärke des Optikrohrs geringer ist als derjenige des Endoskopkopfs, eignet sich das Optikrohr für einen Stauchungsvorgang besser als der Endoskopkopf für einen Quetschvorgang.

In einer weiteren bevorzugten Ausgestaltung wird das Optikrohr beim Verpressen axial lagefest gehalten, und es wird eine axiale Kraft in Richtung eines distalen Endbereichs des Optikrohrs auf den proximalen Endbereich des Optikrohrs ausgeübt.

Diese Maßnahme hat den Vorteil, dass das Verpressen des Optikrohrs mit dem Endoskopkopf auf einfache Weise dadurch erreicht wird, dass das eingeschobene Optikrohr distalseitig des Endoskopkopfs gehalten wird und gleichzeitig eine axiale Kraft, die in Richtung des distalen Endes des Optikrohrs wirkt, auf dieses beaufschlagt wird. Der proximale Endbereich des Optikrohrs wird dadurch gestaucht und mit dem Endoskopkopf verpresst.

In einer weiteren bevorzugten Ausgestaltung wird die axiale Kraft durch ein Verpresswerkzeug, insbesondere einen Dorn, bereitgestellt.

Diese Maßnahme stellt eine technisch einfache Möglichkeit dar, wie die axiale Kraft auf das Optikrohr erzeugt wird. Im Unterschied zu den bekannten Verfahren ist der Werkzeugaufwand wesentlich geringer.

In einer weiteren bevorzugten Ausgestaltung wird der umfängliche Einstich als vollumfängliche Nut bereitgestellt.

Diese Maßnahme hat den Vorteil, dass aufgrund dieser Formgebung des Einstichs besonders viel Material in den Einstich eingreifen kann. Die Verbindung des Endoskopkopfs mit dem Optikrohr ist daher besonders stabil ausgebildet.

In einer weiteren bevorzugten Ausgestaltung wird der distale Endbereich des Endoskopkopfs mit zumindest einer umfänglich begrenzten Ausbuchtung bereitgestellt, in die beim Verpressen Material des Optikrohrs formschlüssig einfließt.

Diese Maßnahme hat den Vorteil, dass das in die umfänglich begrenzte Ausbuchtung eingeschobene Material verhindert, dass sich das Optikrohr relativ zum Endoskopkopf um seine Längsachse verdrehen kann. Hierdurch ergibt sich auf einfache Weise eine Verdrehsicherung des Optikrohrs.

In einer weiteren bevorzugten Ausgestaltung wird zusätzlich ein proximales Ende des Optikrohrs trompetenartig aufgeweitet.

Diese Maßnahme hat den Vorteil, dass das proximale Ende des Optikrohrs von der Längsachse des Optikrohrs aus gesehen wegweist. Werden während einer weiteren Montage Lichtleiter durch das Optikrohr und den Endoskopkopf eingebracht, wird vorteilhafterweise verhindert, dass diese beschädigt werden, bspw. knicken oder brechen.

In einer weiteren bevorzugten Ausgestaltung wird der distale Endbereich des Endoskopkopfs nach dem Verpressen mit dem proximalen Endbereich des Optikrohrs verklebt.

Diese Maßnahme hat den Vorteil, dass das Endoskop gegenüber äußeren Einflüssen, wie z.B. Wasser, abgedichtet wird. Der Klebstoff muss aber nicht wie bei den bekannten Verfahren die Haltekraft für die feste Verbindung zwischen Optikrohr und Endoskopkopf aufbringen, so dass die Klebstelle keine Sollbruchstelle darstellt.

Weitere Vorteile und Merkmale ergeben sich aus der nachfolgenden Beschreibung und der beigefügten Zeichnung.

Es versteht sich, dass die vorstehend genannten und die nachfolgend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele im Zusammenhang mit der beiliegenden Zeichnung näher beschrieben und erläutert.
Es zeigen:
- Fig. 1 eine: Seitenansicht eines Endoskops;
- Fig. 2: ein Optikrohr (ausschnittsweise) und einen Endoskopkopf im Längsschnitt zu Beginn eines Verfahrens zum Herstellen des Endoskops;
- Fig.3: das Optikrohr und den Endoskopkopf in Fig. 2 in einem weiteren Verfahrensschritt des Herstellungsverfahrens;
- Fig. 4: den Endoskopkopf und das Optikrohr in Fig. 3 mit einem Verpresswerkzeug in einem weiteren Verfahrensschritt des Herstellungsverfahrens;
- Fig. 5: den Endoskopkopf und das Optikrohr sowie das Verpresswerkzeug in Fig. 4 in einem weiteren Verfahrensstadium des Herstellungsverfahrens;
- Fig. 6: den Endoskopkopf, das Optikrohr und das Verpresswerkzeug in Fig. 5 in einem weiteren Verfahrensstadium des Herstellungsverfahrens;
- Fig. 7: den Endoskopkopf und das Optikrohr in Fig. 6 und ein weiteres Verpresswerkzeug in einem weiteren Verfahrensstadium des Herstellungsverfahrens;
- Fig. 8: den Endoskopkopf, das Optikrohr und das weitere Verpresswerkzeug in Fig. 7 in einem weiteren Verfahrensschritt des Herstellungsverfahrens; und
- Fig. 9: den Endoskopkopf in einem Querschnitt entlang der Linie I-I in Fig. 8.

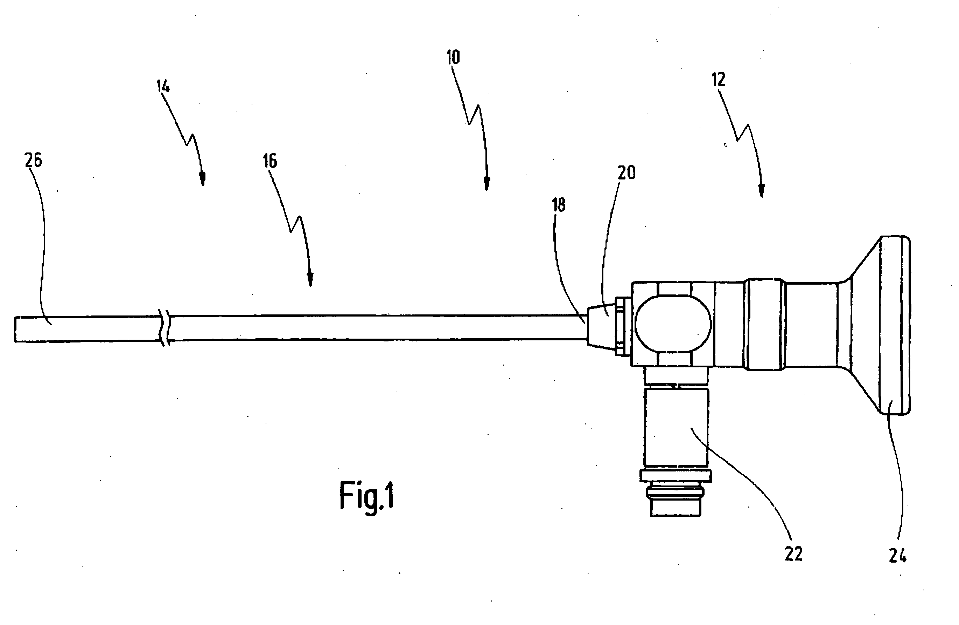

In Fig. 1 ist ein mit dem allgemeinen Bezugszeichen 10 versehenes Endoskop dargestellt. Das Endoskop 10 weist einen Endoskopkopf 12 und einen Endoskopschaft 14 auf.

Ein derartiges Endoskop 10 wird bspw. in der minimal-invasiven Chirurgie zur Untersuchung von Körperhohlräumen oder Hohlorganen verwendet. Hierbei wird das Endoskop 10 distalseitig in eine Körperöffnung eingeführt, so dass zumindest der Endoskopkopf 12 außerhalb des Körpers verbleibt.

Der Endoskopschaft 14 weist ein langerstrecktes Optikrohr 16 auf, dessen proximaler Endbereich 18 mit einem distalen Endbereich 20 des Endoskopkopfs 12 verbunden ist. Das Optikrohr 16 ist vorzugsweise als zylinderförmiger Hohlzylinder mit geringer Wandstärke ausgebildet.

Am Endoskopkopf 12 sind ein Anschluss 22 für eine externe Beleuchtungsquelle und eine Augenmuschel 24 angeordnet. Im Endoskop 10 ist ferner ein Lichtleitsystem aufgenommen, das sich ausgehend von dem Anschluss 22 zu einem distalen Endbereich 26 des Optikrohrs 16 erstreckt und aus Lichtleitfasern gefertigt ist. Das Lichtleitsystem wird zur Beleuchtung eines Operationsgebietes innerhalb der Körperöffnung verwendet. Ferner ist in dem Endoskop 10 ein Bildgebungssystem aufgenommen, das sich von der Augenmuschel 24 bis zu dem distalen Endbereich 26 des Optikrohrs 16 erstreckt. Das Bildgebungssystem kann Lichtleitfasern, Stablinsen oder auch einen Bildsensor mit elektrischer Signalübertragung aufweisen.

Das Endoskop 10 ist vorzugsweise starr ausgebildet, wobei das Optikrohr 16 aus nicht biegsamen Materialien, wie beispielsweise Stahl oder Metall, gefertigt ist.

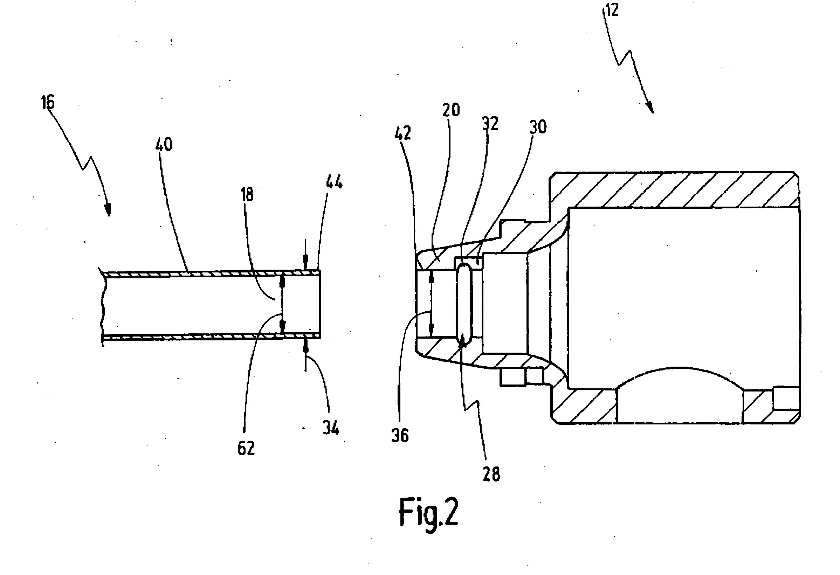

Bei einem Verfahren zum Herstellen des Endoskops 10 wird in einem ersten Verfahrensschritt das Optikrohr 16 sowie der Endoskopkopf 12 bereitgestellt (siehe Fig. 2). Der Endoskopkopf 12 weist innenseitig an seinem distalen Endbereich 20 einen umfänglichen Einstich 28 sowie zumindest eine umfänglich begrenzte Ausbuchtung 30 auf.

Der Einstich 28 ist vorzugsweise als vollumfängliche Nut 32 in dem distalen Endbereich 20 des Endoskopkopfs 12 ausgebildet. Die Ausbuchtung 30 kann entweder direkt an einem Teil des Einstichs 28 angeordnet oder von diesem räumlich beabstandet in dem distalen Endbereich 20 des Endoskopkopfs 12 ausgebildet sein. Ferner kann die Ausbuchtung 30 in radialer Richtung des distalen Endbereichs 20 des Endoskopkopfs 12 gesehen gegenüber dem Einstich 28 vertieft sein.

Ein Außendurchmesser 34 des Optikrohrs 16 ist derart bemessen, dass er geringfügig kleiner als ein Innendurchmesser 36 des distalen Endbereichs 20 des Endoskopkopfs 12 ist.

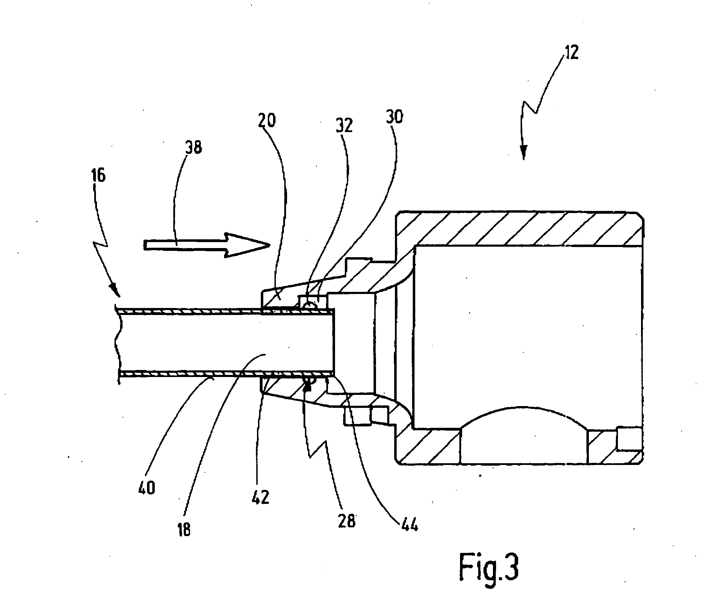

Wie in Fig. 3 dargestellt, wird in einem weiteren Verfahrensschritt der proximale Endbereich 18 des Optikrohrs 16 entlang einer Richtung eines Pfeils 38 in den distalen Endbereich 20 des Endoskopkopfs 12 eingeschoben. Hierbei berührt eine Außenfläche 40 des Optikrohrs 16 eine Innenfläche 42 des Endoskopkopfs 12 oder die Außenfläche 40 des Optikrohrs 16 ist geringfügig beabstandet zu der Innenfläche 42 des distalen Endbereichs 20 des Endoskopfkopfs 12. Ferner wird der proximale Endbereich 18 des Optikrohrs 16 so weit in den distalen Endbereich 20 des Endoskopkopfs 12 eingeschoben, dass zumindest ein proximales Ende 44 des Optikrohrs 16 über den Einstich 28 und die Ausbuchtung 30 hinausreicht.

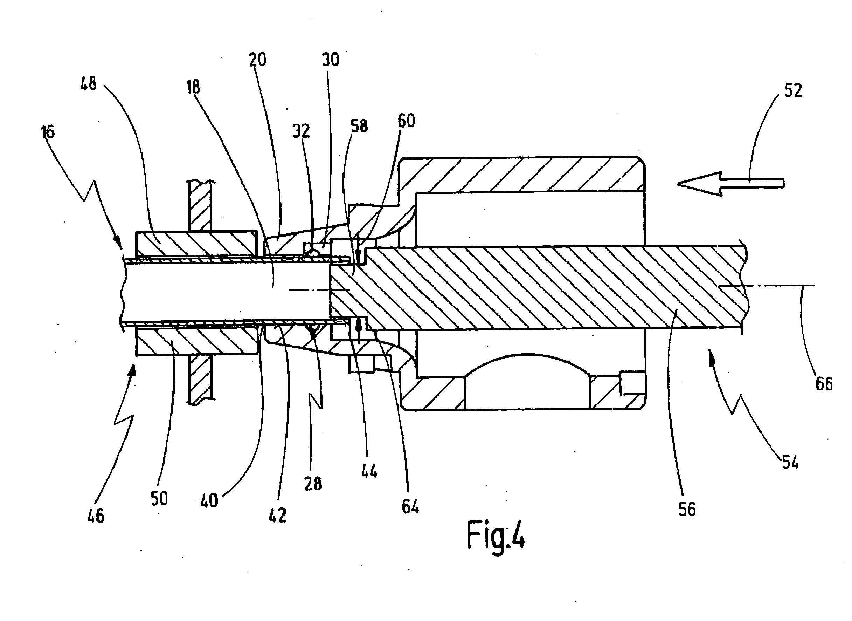

Fig. 4-8 zeigen das Verpressen des distalen Endbereichs 20 des Endoskopkopfs 12 mit dem proximalen Endbereich 18 des Optikrohrs 16.

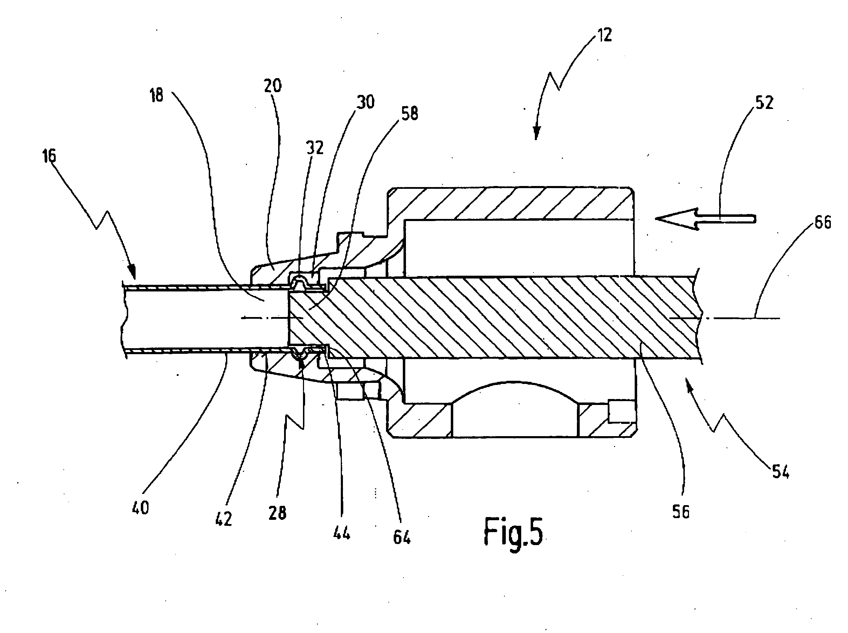

Das Optikrohr 16, dessen proximaler Endbereich 18 in dem distalen Endbereich 20 des Endoskopkopfs 12 eingeschoben ist, wird axial lagefest gegenüber Zug- und Druckkräften, die in axialer Richtung des Optikrohrs 16 weisen, gehalten (siehe Fig. 4). Hierzu wird das Optikrohr 16 in eine Haltevorrichtung 46 eingespannt. Solch eine Haltevorrichtung 46 kann bspw. zwei Backen 48, 50 aufweisen, die an der Außenfläche 40 des eingeschobenen Optikrohrs 16 distalseitig des distalen Endbereichs 20 des Endoskopkopfs 12 angreifen. Die beiden Backen 48, 50 berühren entweder den distalen Endbereich 20 des Endoskopkopfs 12 oder sie sind zu diesem geringfügig beabstandet. Die Backen 48, 50 der Haltevorrichtung 46 sind in Fig. 4 dargestellt, während sie übersichtshalber in Fig. 5-8 nicht gezeigt sind.

Es wird in Richtung eines Pfeils 52 eine axiale Kraft auf den proximalen Endbereich 18 des Optikrohrs 16 ausgeübt, Die axiale Kraft wird mittels eines Verpresswerkzeugs 54 erzeugt, das proximalseitig durch den Endoskopkopf 12 in das Optikrohr 16 eingeführt wird.

Das Verpresswerkzeug 54 kann vorzugsweie als zylinderförmiger Dorn 56 ausgebildet sein. Das Verpresswerkzeug 54 verjüngt sich an einem distalen Ende 58 stufenförmig, so dass ein Außendurchmesser 60 des distalen Endes 58 des Verpresswerkzeugs 54 geringfügig kleiner als ein Innendurchmesser 62 des Optikrohrs 16 ausgebildet ist. Ferner weist das Verpresswerkzeug 54 im Bereich der stufenförmigen Verjüngung eine vorzugsweise vollumfängliche Planfläche 64 auf, die quer zu einer Längsachse 66 des Verpresswerkzeugs 54 ausgebildet ist, an der das proximale Ende 44 des Optikrohrs 16 vollumfänglich zur Anlage kommt. Vorzugsweise ist das distale Ende 58 des Verpresswerkzeugs 54 derart lang ausgebildet, dass das Verpresswerkzeug 54 in einem vollständig eingeschobenen Zustand, d.h. wenn die Planfläche 64 das proximale Ende 44 des Optikrohrs 16 berührt, über den Einstich 28 und die Ausbuchtung 30 reicht.

Wie in Fig. 5 dargestellt, wird der proximale Endbereich 18 des Optikrohrs 16 derart durch die axiale Kraft gestaucht, dass gestauchtes Material radial nach außen verschoben wird. Dies ist insbesondere der Fall, wenn das distale Ende 58 des Verpresswerkzeugs 54 über den Einstich 28 und die Ausbuchtung 30 reicht und somit ein radial nach innen gerichtetes Verschieben des gestauchten Materials verhindert, Hierbei greift Material des proximalen Endbereichs 18 des Optikrohrs 16 formschlüssig in den Einstich 28 ein, während das Optikrohr 16 mit dem Endoskopkopf 12 verpresst wird. Das Eingreifen des Materials des Optikrohrs 16 in den Einstich 28 ermöglicht eine sichere axiale Lagefixierung des Optikrohrs 16 bezüglich des Endoskopkopfs 12.

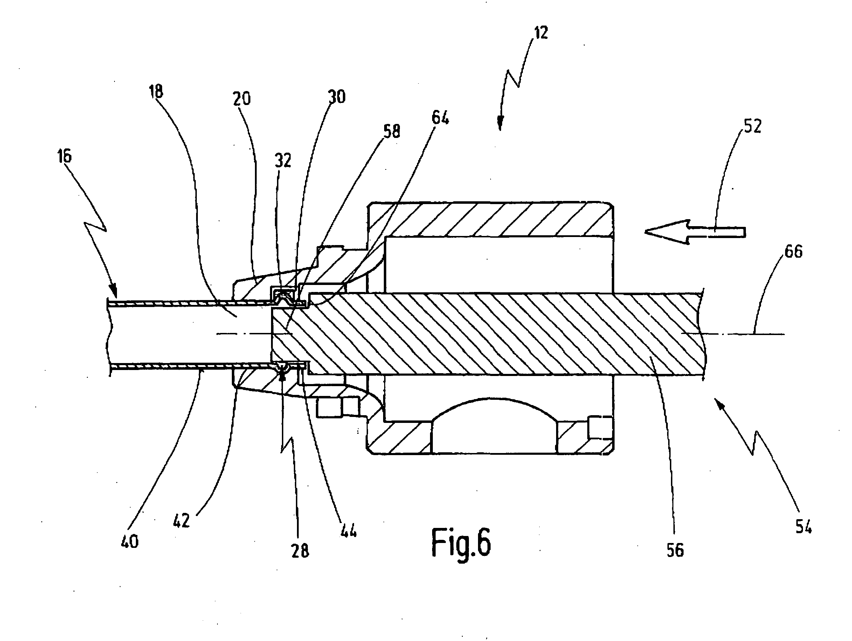

Durch das Verpressen greift ferner Material des Optikrohrs 16 in die Ausbuchtung 30 ein (siehe Fig. 6). Hierdurch wird verhindert, dass sich das Optikrohr 16 relativ zum Endoskopkopf 12 um seine Längsachse verdrehen kann.

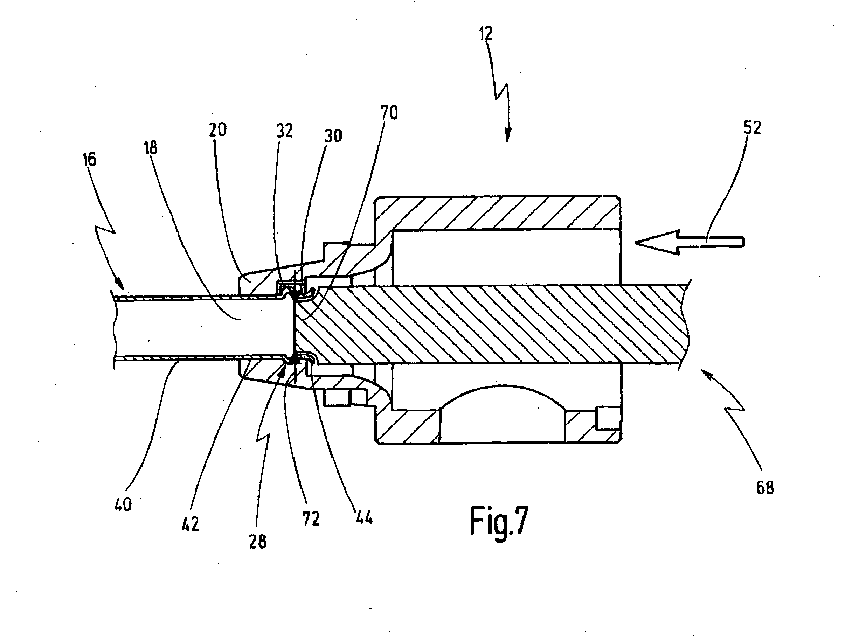

Wie in Fig. 7 dargestellt, wird zusätzlich das proximale Ende 44 des Optikrohrs 16 trompetenartig aufgeweitet, so dass sich der Außendurchmesser 34 des Optikrohrs 16 an dieser Stelle vergrößert. Das proximale Ende 44 des Optikrohrs 16 weist von einer Längsachse des Optikrohrs 16 aus gesehen nach außen weg. Hierzu wird das Verpresswerkzeug 54 aus dem Optikrohr 16 und dem Endoskopkopf 12 entnommen. Es wird ein weiteres Verpresswerkzeug 68 in Richtung des Pfeils 52 durch den Endoskopkopf 12 in das verpresste Optikrohr 16 eingeschoben, mit dem eine axiale Kraft auf den proximalen Endbereich 18 des Optikrohrs 16 erzeugt wird.

Das weitere Verpresswerkzeug 68 verjüngt sich ebenfalls distalseitig, wobei die Verjüngung nicht stufenförmig ausgebildet ist, sondern sich über einen konkav gekrümmten Teilbereich eines distalen Endes 70 erstreckt. Ein kleinster Außendurchmesser 72 des distalen Endes 70 des weiteren Verpresswerkzeugs 68 ist ebenfalls geringfügig kleiner als der Innendurchmesser 62 des Optikrohrs 16 ausgebildet. Das sich verjüngende distale Ende 70 des weiteren Verpresswerkzeugs 68 dehnt das proximale Ende 44 des Optikrohrs 16 derart aus, dass sich eine Form des proximalen Endes 44 des Optikrohrs 16 einer Form des distalen Endes 70 des weiteren Verpresswerkzeugs 68 anpasst. Das Verpresswerkzeug 54 und das Verpresswerkzeug 68 können auch als ein Verpresswerkzeug ausgebildet sein, mit dem der proximale Endbereich 18 des Optikrohrs 16 sowohl gestaucht als auch das proximale Ende 44 des Optikrohrs 16 aufgeweitet werden kann.

Das aufgeweitete proximale Ende 44 des Optikrohrs 16 verhindert bei einem späteren Einführen von Lichtleitfasern in das Endoskop 10 eine Beschädigung letzterer.

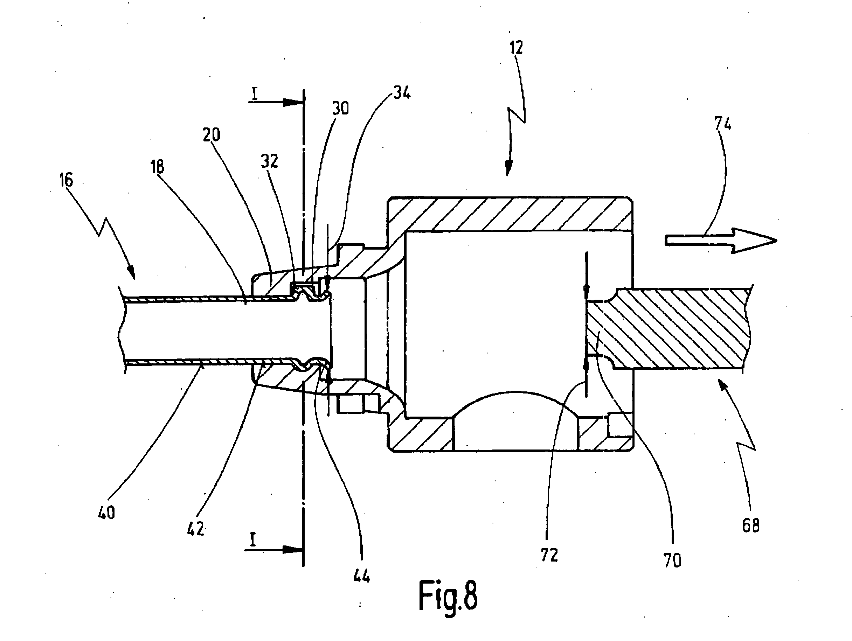

Danach wird das weitere Verpresswerkzeug 68 in Richtung eines Pfeils 74 aus dem Endoskopkopf 12 entnommen (siehe Fig. 8).

Der proximale Endbereich 18 des Optikrohrs 16 wird nach dem Verpressen ferner mit dem distalen Endbereich 20 des Endoskopkopfs 12 verklebt. Hierzu wird ein Klebstoff auf die Außenfläche 40 des Optikrohrs 16 und/oder auf die Innenfläche 42 des distalen Endbereichs 20 des Endoskopkopfs 12 aufgebracht- Das Verkleben des Optikrohrs 16 mit dem Endoskopkopf 12 dient zur Abdichtung der Verbindungsstelle beider Endoskopteile gegenüber äußeren Einflüssen, wie bspw. Wasser.

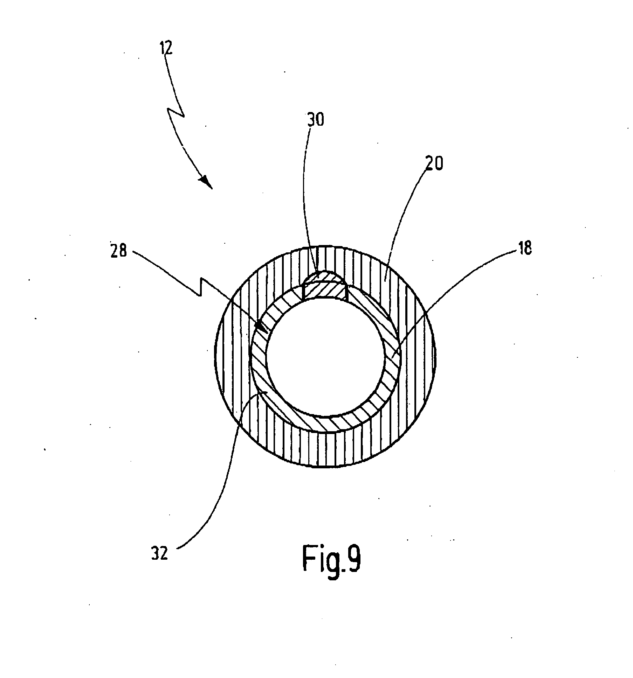

Fig. 9 zeigt eine Querschnittszeichnung des distalen Endbereichs 20 des Endoskopkopfs 12 entlang der Linie I-I in Fig. 8. Der proximale Endbereich 18 des Optikrohrs 16 ist mit dem distalen Endbereich 20 des Endoskopkopfs 12 verpresst. Material des Optikrohrs 16 greift formschlüssig in den als vollumfängliche Nut 32 ausgebildeten Einstich 28 und in die Ausbuchtung 30 ein.

## Patentansprüche

1. Verfahren zum Herstellen eines Endoskops (10), wobei das Endoskop (10) einen Endoskopkopf (12) und ein Optikrohr (16) aufweist, mit den Schritten
- Bereitstellen des Endoskopkopfs (12),
- Bereitstellen des Optikrohrs (16),
- Einschieben eines proximalen Endbereichs (18) des Optikrohrs (16) in einen distalen Endbereich (20) des Endoskopkopfs (12),
**dadurch gekennzeichnet, dass** der distale Endbereich (20) des Endoskopkopfs (12) mit dem proximalen Endbereich (18) des Optikrohrs (16) verpresst wird, und dass der distale Endbereich (20) des Endoskopkopfs (12) innenseitig mit einem umfänglichen Einstich (28) bereitgestellt wird, in den beim Verpressen Material des Optikrohrs (16) formschlüssig einfließt.

2. Verfahren nach Anspruch 1, **dadurch gekennzeichnet, dass** der proximale Endbereich (18) des Optikrohrs (16) durch Stauchen verpresst wird.

3. Verfahren nach Anspruch 1 oder 2, **dadurch gekennzeichnet, dass** das Optikrohr (16) beim Verpressen axial lagefest gehalten wird und eine axiale Kraft in Richtung eines distalen Endbereichs (26) des Optikrohrs (16) auf den proximalen Endbereich (18) des Optikrohrs (16) ausgeübt wird.

4. Verfahren nach Anspruch 3, **dadurch gekennzeichnet, dass** die axiale Kraft durch ein Verpresswerkzeug (54), insbesondere einen Dorn (56), bereitgestellt wird.

5. Verfahren nach einem der Ansprüche 1 bis 4, **dadurch gekennzeichnet, dass** der umfängliche Einstich (28) als vollumfängliche Nut (32) bereitgestellt wird.

6. Verfahren nach einem der Ansprüche 1 bis 5, **dadurch gekennzeichnet, dass** der distale Endbereich (20) des Endoskopkopfs (12) mit zumindest einer umfänglich begrenzten Ausbuchtung (30) bereitgestellt wird, in die beim Verpressen Material des Optikrohrs (16) formschlüssig einfließt.

7. Verfahren nach einem der Ansprüche 1 bis 6, **dadurch gekennzeichnet, dass** zusätzlich ein proximales Ende (44) des Optikrohrs (16) trompetenartig aufgeweitet wird.

8. Verfahren nach einem der Ansprüche 1 bis 7, **dadurch gekennzeichnet, dass** der distale Endbereich (20) des Endoskopkopfs (12) nach dem Verpressen mit dem proximalen Endbereich (18) des Optikrohrs (16) verklebt wird.

9. Endoskop, mit einem Endoskopkopf (12) und einem Optikrohr (16), **dadurch gekennzeichnet, dass** ein proximaler Endbereich (18) des Optikrohrs (16) mit einem distalen Endbereich (20) des Endoskopkopfs (12) verpresst ist, und dass der distale Endbereich (20) des Endoskopkopfs (12) innenseitig einen umfänglichen Einstich (28) aufweist, in den Material des Optikrohrs (16) formschlüssig eingreift.

10. Endoskop nach Anspruch 9, **dadurch gekennzeichnet, dass** der umfängliche Einstich (24) als.vollumfängliche Nut (32) ausgebildet ist.

11. Endoskop nach Anspruch 9 oder 10, **dadurch gekennzeichnet, dass** der distale Endbereich (20) des Endoskopkopfs (12) innenseitig zumindest eine umfänglich begrenzte Ausbuchtung (30) aufweist, in die Material des Optikrohrs (16) formschlüssig eingreift.

12. Endoskop nach einem der Ansprüche 9 bis 11, **dadurch gekennzeichnet, dass** ein proximales Ende (44) des Optikrohrs (16) trompetenartig aufgeweitet ist.

## Claims

1. A method for producing an endoscope (10), the endoscope (10) comprising an endoscope head (12) and an optic tube (16), said method comprising the steps of
- providing the endoscope head (12),
- providing the optic tube (16),
- pushing a proximal end area (18) of the optic tube (16) into a distal end area (20) of the endoscope head (12),
**characterized in that** the distal end area (20) of the endoscope head (12) is press-fitted with the proximal end area (18) of the optic tube (16), and that the distal end area (20) of the endoscope head (12) is provided on its inside with a circumferential recess (28) into which material of the optic tube (16) flows with a form fit during the pressing operation.

2. The method of Claim 1, **characterized in that** the proximal end area (18) of the optic tube (16) is press-fitted by axial compressing.

3. The method of Claim 1 or 2, **characterized in that** the optic tube (16) is held in an axially fixed position during the pressing operation, and an axial force in the direction of a distal end area (26) of the optic tube (16) is exerted on the proximal end area (18) of the optic tube (16).

4. The method of Claim 3, **characterized in that** the axial force is provided by a pressing tool (54), in particular a mandrel (56).

5. The method of anyone of Claim 1 through 4, **characterized in that** the circumferential recess (28) is provided as a groove (32) extending about the full circumference.

6. The method of anyone of Claims 1 through 5, **characterized in that** the distal end area (20) of the endoscope head (12) is provided with at least one circumferentially limited cavity (30) into which material of the optic tube (16) flows with a form fit during the pressing operation.

7. The method of anyone of Claims 1 through 6, **characterized in that** a proximal end (44) of the optic tube (16) is additionally widened in a trumpet shape.

8. The method of anyone of Claims 1 through 7, **characterized in that** the distal end area (20) of the endoscope head (12) is adhesively bonded to the proximal end area (18) of the optic tube (16) after the pressing operation.

9. An endoscope, comprising an endoscope head (12) and an optic tube (16), **characterized in that** a proximal end area (18) of the optic tube (16) is press-fitted with a distal end area (20) of the endoscope head (12), and that the distal end area (20) of the endoscope head (12) has, on its inside, a circumferential recess (28) into which material of the optic tube (16) engages with a form fit.

10. The endoscope of Claim 9, **characterized in that** the circumferential recess (24) is designed as a groove (32) extending about the full circumference.

11. The endoscope of Claim 9 or 10, **characterized in that** the distal end area (20) of the endoscope head (12) has, on its inside, at least one circumferentially limited cavity (30) into which material of the optic tube (16) engages with a form fit.

12. The endoscope of anyone of Claims 9 through 11, **characterized in that** a proximal end (44) of the optic tube (16) is widened in a trumpet shape.

## Revendications

1. Procédé servant à fabriquer un endoscope (10), sachant que l'endoscope (10) présente une tête d'endoscope (12) et un tube optique (16), ledit procédé comportant les étapes suivantes consistant à :
- fournir une tête d'endoscope (12),
- fournir le tube optique (16),
- insérer par coulissement une zone d'extrémité proximale (18) du tube optique (16) dans une zone d'extrémité distale (20) de la tête d'endoscope (12),
**caractérisé en ce que** la zone d'extrémité distale (20) de la tête d'endoscope (12) est pressée avec la zone d'extrémité proximale (18) du tube optique (16), et **en ce que** la zone d'extrémité distale (20) de la tête d'endoscope (12) est fournie côté intérieur avec une encoche (28) périphérique, dans laquelle le matériau du tube optique (16) s'écoule par complémentarité de forme par pressage.

2. Procédé selon la revendication 1, **caractérisé en ce que** la zone d'extrémité proximale (18) du tube optique (16) est pressée par refoulement.

3. Procédé selon la revendication 1 ou 2, **caractérisé en ce que** le tube optique (16) est maintenu fixement dans une position de manière axiale lors du pressage, et **en ce qu'**une force axiale est exercée en direction d'une zone d'extrémité distale (26) du tube optique (16) sur la zone d'extrémité proximale (18) du tube optique (16).

4. Procédé selon la revendication 3, **caractérisé en ce que** la force axiale est fournie par un outil de pressage (54), en particulier par un mandrin (56).

5. Procédé selon l'une quelconque des revendications 1 à 4, **caractérisé en ce que** l'encoche (28) périphérique est fournie comme une rainure (32) sur toute la surface.

6. Procédé selon l'une quelconque des revendications 1 à 5, **caractérisé en ce que** la zone d'extrémité distale (20) de la tête d'endoscope (12) est fournie avec au moins un renflement (30) délimité de manière périphérique, dans lequel le matériau du tube optique (16) s'écoule par complémentarité de forme lors du pressage.

7. Procédé selon l'une quelconque des revendications 1 à 6, **caractérisé en ce qu'**en outre une extrémité proximale (44) du tube optique (16) est élargie de manière à présenter une forme de trompette.

8. Procédé selon l'une quelconque des revendications 1 à 7, **caractérisé en ce que** la zone d'extrémité distale (20) de la tête d'endoscope (12) est collée après le pressage à la zone d'extrémité proximale (18) du tube optique (16).

9. Endoscope comportant une tête d'endoscope (12) et un tube optique (16), **caractérisé en ce qu'**une zone d'extrémité proximale (18) du tube optique (16) est pressée avec une zone d'extrémité distale (20) de la tête d'endoscope (12), et **en ce que** la zone d'extrémité distale (20) de la tête d'endoscope (12) présente côté intérieur une encoche (28) périphérique, dans laquelle le matériau du tube optique (16) vient en prise par complémentarité de forme.

10. Endoscope selon la revendication 9, **caractérisé en ce que** l'encoche (24) périphérique est réalisée comme une rainure (32) sur toute la surface.

11. Endoscope selon la revendication 9 ou 10, **caractérisé en ce que** la zone d'extrémité distale (20) de la tête d'endoscope (12) présente côté intérieur au moins un renflement (30) délimité de manière périphérique, dans lequel le matériau du tube optique (16) vient en prise par complémentarité de forme.

12. Endoscope selon l'une quelconque des revendications 9 à 11, **caractérisé en ce qu'**une extrémité proximale (44) du tube optique (16) est élargie de manière à présenter une forme de trompette.
